# FASCICULE DE BREVET EUROPEEN

(11) **EP 1 303 225 B1**
(45) Date de publication et mention de la délivrance du brevet: **25.05.2005**
(21) Numéro de dépôt: 01958176.8
(22) Date de dépôt: 25.07.2001
(51) Int. Cl.: A61B 17/70

(54) **ELEMENT DE LIAISON SOUPLE POUR LA STABILISATION DU RACHIS**
FLEXIBLES VERBINDUNGSSTÜCK ZUR STABILISIERUNG DER WIRBELSÄULE
FLEXIBLE LINKING PIECE FOR STABILISING THE SPINE

(30) Priorité: 25.07.2000 FR 0009706
(43) Date de publication de la demande: 23.04.2003
(73) Titulaire: Spine Next, La Cité Mondiale, 33000 Bordeaux (FR)
(72) Inventeur: LE COUEDIC, Régis, F-33000 Bordeaux (FR); PASQUET, Denis, F-33600 Pessac (FR)
(74) Mandataire: Dronne, Guy
(86) Numéro de dépôt international: PCT/FR2001/002426
(87) Numéro de publication internationale: WO 2002/007622

(56) Documents cités:
- FR-A- 2 676 911
- FR-A- 2 730 405
- FR-A- 2 755 844
- US-A- 5 002 576
- US-A- 5 375 823
- PATENT ABSTRACTS OF JAPAN vol. 1999, no. 01, 29 janvier 1999 (1999-01-29) & JP 10 277070 A (SANO SHIGEO;ITO KAORU), 20 octobre 1998 (1998-10-20)

## Description

La présente invention concerne une pièce de liaison destinée à maintenir un espacement entre au moins deux éléments d'ancrage reliés par ladite pièce de liaison.

Des domaines d'application de l'invention sont notamment la stabilisation et les arthrodèses de segments de la colonne vertébrale dans les pathologies dégénératives du rachis.

Des systèmes de stabilisation de la colonne vertébrale maintenant au moins deux vertèbres consécutives au moyen d'éléments d'ancrage fixés dans lesdites vertèbres et reliés par des tiges de liaison rigides, sont bien connus. De tels systèmes sont généralement accouplés de façon à relier deux vertèbres consécutives par deux tiges sensiblement parallèles fixées de chaque côté des apophyses épineuses. Les éléments d'ancrage vissés dans la partie postérieure des vertèbres traversent les pédicules et une partie substantielle des corps vertébraux de façon à obtenir une liaison fixe et durable dans le temps.

Ces systèmes de stabilisation sont couramment utilisés pour consolider plusieurs vertèbres consécutives. Ainsi, les vertèbres sont-elles reliées les unes aux autres au moyen de tiges rigides sur une longueur substantielle de la colonne vertébrale. De tels montages permettent d'assurer correctement le maintien des vertèbres les unes par rapport aux autres ; en revanche, ils raidissent considérablement la flexion du rachis et interdisent la mobilité relative des vertèbres les unes par rapport aux autres selon l'axe longitudinal du rachis. Il a été démontré qu'un système de stabilisation plus flexible et relativement déformable, conférant aux vertèbres une plus grande mobilité relative entre elles, était bénéfique dans le cas de certaines pathologies. FR-A1 2 730 405 divulgue un dispositif de liaison omnidirectionelle selon le préambule de la revendication 1.

Un premier objet de la présente invention est de fournir une pièce de liaison apte à maintenir espacés des éléments d'ancrage existants, tout en permettant une mobilité relative desdits éléments d'ancrage.

Pour atteindre ce but, conformément à l'invention définie dans la revendication 1, la pièce de liaison destinée à maintenir un espacement entre au moins deux éléments d'ancrage vissés dans des vertèbres, comprend au moins : deux parties rigides formant tiges, constituées dans un premier matériau, présentant chacune une première portion de fixation apte à être fixée dans un élément d'ancrage et une deuxième portion de solidarisation, lesdites tiges étant situées dans le prolongement l'une de l'autre et lesdites portions de solidarisation en regard l'une de l'autre, et, un organe de liaison intégralement constitué dans un second matériau de plus grande déformabilité élastique que ledit premier matériau, reliant lesdites parties rigides par leurs dites portions de solidarisation en regard l'une de l'autre de façon que ladite pièce de liaison soit apte à être déformée élastiquement, par quoi les vertèbres, maintenues espacées l'une de l'autre, sont mobiles l'une par rapport à l'autre.

Ainsi, une caractéristique de la pièce de liaison réside dans le mode de solidarisation des deux parties rigides ensemble par un organe de liaison élastiquement déformable qui confère aux parties rigides, sous contraintes, une mobilité relative dont les forces de réaction aux contraintes sont proportionnelles, dans certaines limites, à la déformation de l'organe de liaison. De la sorte, la pièce de liaison est susceptible de fléchir sous l'action de contraintes dont les directions ne sont pas parallèles à l'axe de la pièce ; elle est également susceptible d'être allongée ou contractée sous l'action de forces opposées parallèles à l'axe de la pièce.

En conséquence, les deux éléments d'ancrage, reliés par la pièce de liaison au repos, de façon que les portions fixables soient solidaires des éléments d'ancrage, sont susceptibles d'être déplacés l'un par rapport à l'autre par des forces dont les intensités sont proportionnelles à la valeur du déplacement.

Préférentiellement, lesdites parties rigides sont reliées mécaniquement entre elles par un seul organe de liaison réalisant l'intégralité de ladite liaison mécanique. De la sorte, un seul organe assure à la fois la liaison entre les parties rigides et le contrôle du déplacement relatif de ces parties rigides entre elles. En outre, selon un mode particulier de mise en oeuvre, ledit organe de liaison est intégralement constitué dans un seul second matériau de façon à simplifier le montage et à lui procurer des propriétés mécaniques homogènes.

Avantageusement, la pièce de liaison selon l'invention, comprend n parties rigides entre lesquelles sont interposées n-1 organes de liaison selon l'axe longitudinal de ladite pièce, chaque partie rigide située entre deux organes de liaison comportant une première portion de fixation et deux deuxièmes portions de solidarisation, chacune desdites deuxièmes portions de solidarisation étant située de chaque côté de ladite première portion de fixation, lesdites deuxièmes portions de solidarisation étant reliées auxdits deux organes de liaison, et en ce que les parties rigides situées aux deux extrémités de ladite pièce présente une seule deuxième portion de solidarisation reliée aux organes de liaison, par quoi ladite pièce est apte à relier n éléments d'ancrage.

Ainsi, selon cette caractéristique, la pièce de liaison maintient un espacement entre tous les éléments d'ancrage qu'elle relie, ces derniers étant susceptibles d'être fixés chacun dans une vertèbre, de façon à constituer un alignement. Chaque partie rigide est fixée à un élément d'ancrage et entre chaque élément d'ancrage un organe de liaison relie les deux portions de solidarisation. De la sorte, une pluralité de vertèbres est stabilisée avec une seule pièce de liaison unique, ce qui permet de diminuer le temps de montage de l'ensemble du système de stabilisation et par conséquent le temps opératoire. En outre, cette caractéristique de la pièce de liaison permet d'assurer une stabilisation sur plusieurs vertèbres consécutives en les reliant entre elles, tout en leur conférant une flexibilité et une compressibilité longitudinale relative, importantes.

Selon un mode préféré de mise en oeuvre de l'invention lesdites portions de solidarisation desdites parties rigides que ledit organe de liaison relie présentent une paroi de solidarisation sur laquelle ledit organe de liaison est apte à adhérer. Ainsi, aucune pièce de fixation additionnelle n'est nécessaire et les propriétés adhésives du deuxième matériau sur la paroi de solidarisation suffisent à assurer la liaison.

Selon un mode particulier de mise en oeuvre de l'invention, ladite paroi de solidarisation présente des évidements aptes à coopérer avec des aspérités dudit organe de liaison de façon à augmenter la surface de contact entre ladite paroi et ledit organe.

On comprend en effet que le fait de ménager des évidements dans une paroi accroît la surface de cette paroi, ce qui augmente la surface de contact entre les deux matériaux si l'un des matériaux peut être moulé sur la paroi de l'autre. L'augmentation de la surface de contact induit l'augmentation des forces de liaison entre ledit organe de liaison et lesdites portions de solidarisation. En outre, les forces de frottement statique du matériau de la pièce de liaison sur lesdits deux éléments sont corrélativement augmentées et ces forces s'additionnent aux forces de liaison.

Avantageusement, ledit deuxième matériau dudit organe de liaison est constitué d'un corps obtenu par polymérisation. De la sorte, l'organe de liaison peut facilement être moulé à chaud sur les parois de solidarisation si le matériau est polymérisé au préalable, ou il peut être constitué in situ si la vitesse de polymérisation des monomères constituant ledit deuxième matériau est suffisamment faible pour disposer du temps nécessaire à la réalisation de l'assemblage.

Selon un mode préféré de mise en oeuvre de l'invention, ledit premier matériau desdites parties rigides est un alliage de titane. Ainsi, il est aisé de ménager des évidements dans ladite paroi de solidarisation sur laquelle ledit organe de liaison est apte à adhérer.

Selon un autre mode préféré de mise en oeuvre de l'invention, la section desdites parties rigides formant tiges est circulaire, ce qui facilite la réalisation de la pièce. Par ailleurs, dans le cas où des tiges de liaison de section circulaire conformes à celles de l'art antérieur sont remplacées par des pièces de liaison conformes à l'invention sans avoir besoin de remplacer les éléments d'ancrage, il est nécessaire que lesdites parties rigides comportent des sections identiques aux sections des tiges de liaison de l'art antérieur.

Un deuxième objet de la présente invention est de proposer un système de stabilisation vertébrale destiné à solidariser au moins deux vertèbres, lesdites vertèbres présentant chacune un plan moyen sensiblement perpendiculaire à l'axe du rachis qu'elles forment et une paroi postérieure définissant un plan moyen postérieur dudit rachis, ledit système comportant au moins deux éléments d'ancrage aptes à être fixés chacun dans la paroi postérieure d'une vertèbre de façon que la ligne qui coupe lesdits deux éléments d'ancrage soit sensiblement parallèle audit axe du rachis, ledit système comprenant au moins une pièce de liaison selon l'invention, apte à relier lesdits deux éléments d'ancrage par lesdites deux parties rigides de façon que l'axe de ladite pièce de liaison soit sensiblement parallèle audit axe du rachis, par quoi lesdites vertèbres, reliées dans leur partie postérieure, présentent une mobilité relative selon ledit axe dudit rachis et selon un plan perpendiculaire audit axe du rachis.

D'autres particularités et avantages de l'invention ressortiront à la lecture de la description faite ci-après de modes de réalisation particuliers de l'invention, donnés à titre indicatif mais non limitatif, en référence aux dessins annexés sur lesquels :
- la Figure 1 est une vue schématique en perspective de la pièce de liaison conforme à l'invention,
- la Figure 2 est une vue schématique en coupe axiale de la pièce de liaison conforme à l'invention,
- la Figure 3, est une vue schématique en perspective montrant les éléments d'ancrage reliés par la pièce de liaison, et
- la Figure 4 est une vue schématique latérale en élévation de la colonne vertébrale montrant deux vertèbres consécutives dans lesquelles sont vissés les éléments d'ancrage, lesdits éléments d'ancrage étant reliés par la pièce de liaison conforme à l'invention.

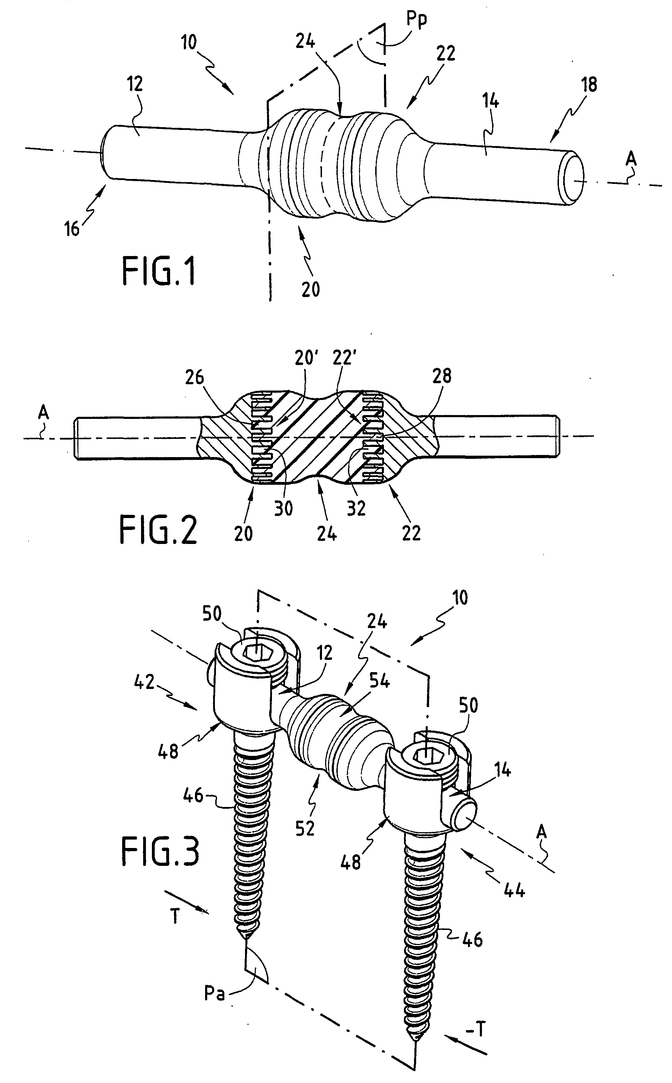

En se référant tout d'abord à la Figure 1 on décrira les différentes parties qui constituent une pièce de liaison selon l'invention.

La pièce de liaison 10 comporte deux parties rigides 12 et 14 de forme cylindrique. Les parties rigides 12, 14 présentent une première portion 16, 18 apte à être fixée et une deuxième portion de solidarisation 20, 22 formant un renflement, les portions de solidarisation 20 et 22 en regard étant reliées entre elles par un organe de liaison 24 de façon que les parties rigides 12 et 14 soient coaxiales. Ainsi, la pièce de liaison 10 présente une symétrie cylindrique d'axe A.

On se référera à la Figure 2 pour décrire le mode de solidarisation des deux parties rigides 12 et 14.

L'organe de liaison 24 est constitué d'un corps, obtenu par polymérisation, du type matière plastique. Ce corps est choisi parmi les matériaux dont la déformabilité élastique est supérieure à celle du matériau desdites parties rigides 12, 14 et surtout dont les propriétés élastiques sont de l'ordre de celles des propriétés des ligaments postérieurs qui maintiennent les différents éléments du rachis.

Les composés organiques du silicium forment des polymères dont les propriétés mécaniques sont susceptibles d'être déterminées par le choix des composés de base, notamment par leur degré de substitution, la nature des substituants et leur poids moléculaire, et dont le comportement élastique est prépondérant par rapport au comportement plastique. Ainsi, ils constituent une famille de matériaux adaptés à la réalisation de la liaison entre lesdites deux parties rigides 12 et 14. En outre, ces polymères sont susceptibles de présenter une forte adhésivité sur les matériaux de composition minérale. Ainsi, l'organe de liaison 24 assure une bonne solidarisation des parties rigides 12, 14 qui sont généralement réalisées en alliage de titane.

Cependant, les matériaux utilisables de type polymères ne sont pas limités aux composés organiques du silicium et tout autre matériau présentant des propriétés comparables pourrait convenir.

Le matériau dudit organe de liaison 24 est apte à adhérer sur les parois de solidarisation 20' et 22' desdites deuxièmes portions de solidarisation 20, 22. Cependant, afin d'accroître cette adhésivité, des évidements 30, 32 sont ménagés dans les parois de solidarisation 20, 22 des deuxièmes portions de solidarisation et sont aptes à coopérer avec des aspérités 26, 28 de l'organe de liaison 24 qui s'insèrent dans les évidements 30, 32.

Cette caractéristique permet, d'une part d'augmenter la surface de contact entre les deux matériaux et ainsi d'augmenter la force de liaison entre eux selon une direction normale à ladite surface de contact, et d'autre part de créer des forces de frottement statique qui s'additionnent à la force d'adhérence.

Une telle liaison est réalisée soit en injectant le polymère à chaud entre les deux parties rigides 12 et 14 maintenues en regard dans un moule, soit en moulant le mélange de monomères à froid entre les deux parties rigides 12 et 14 si la vitesse de réaction est suffisamment lente. Ainsi, les aspérités 26, 28, sont-elles formées in situ, lorsque le polymère en phase liquide ou pâteuse, inséré dans les évidements 26, 28, se solidifie après refroidissement ou après réaction chimique. On comprend que l'organe de liaison 24 est constitué par le polymère interposé entre les parties rigides 12 et 14, plus particulièrement entre les parois de solidarisation 20' et 22' et que, pour le maintenir entre ces parties en regard lorsqu'il est à l'état liquide, les parois du moule doivent nécessairement entourer l'espace qui sépare les deux parties rigides 12, 14 dans leur prolongement.

Selon un mode particulier de réalisation, non représenté, les évidements 30, 32, ménagés dans les parois de solidarisation 20' et 22', débouchent dans la paroi externe des parties rigides 12 et 14 afin que le polymère en phase liquide pénètre entièrement dans les évidements 30, 32 sans que de l'air puisse y être emprisonné. De la sorte, la solidarisation de l'organe de liaison 24 sur les parties rigides 12, 14 est renforcée.

Par ailleurs, les évidements 30, 32, représentés parallèles à l'axe longitudinal de la pièce de liaison sur la Figure 2, sont susceptibles d'être ménagés obliquement par rapport à cet axe longitudinal et/ou non rectiligne. Ces configurations permettent d'accroître les forces de frottement statique du polymère sur les parties rigides, ce qui renforce leur solidarisation.

Après avoir décrit le mode de solidarisation des deux parties rigides, on se référera à nouveau à la Figure 1 pour décrire les mouvements possibles des parties rigides l'une par rapport à l'autre.

Compte tenu de la symétrie cylindrique des parties rigides 12 et 14, et de l'organe de liaison 24, ainsi que de la nature du matériau de l'organe de liaison 24, la pièce de liaison 10 est apte à fléchir, lorsque les deux premières portions fixables sont immobilisées, dans toutes les directions contenues dans un plan Pp perpendiculaire à l'axe A de la pièce de liaison. Le fléchissement de la pièce de liaison 10 entraîne la compression d'un bord de l'organe de liaison 24 et corrélativement l'extension du bord diamétralement opposé tandis que les parties rigides 12 et 14 conservent leur forme. Le matériau de l'organe de liaison 24 étant élastiquement déformable, lorsque les contraintes provoquant le fléchissement cessent, la pièce de liaison 10 retrouve son état initial dans lequel les parties rigides 12 et 14 sont coaxiales.

En outre, les parties rigides 12 et 14 peuvent jouer l'une par rapport à l'autre suivant l'axe longitudinal A dans des directions opposées conduisant à la compression de l'organe de liaison 24 ou à son extension.

Le déplacement relatif des deux parties rigides 12 et 14 peut être effectué dans des directions autres que les directions décrites ci-dessus, mais comme on le décrira plus en détails, la pièce de liaison est principalement sollicitée en flexion, en extension et en compression.

On se référera maintenant à la Figure 3 pour décrire la déformation de la pièce de liaison par rapport aux mouvements relatifs des éléments d'ancrage 42 et 44 illustrés.

On retrouve sur la Figure 3 la pièce de liaison 10 reliant les deux éléments d'ancrage 42 et 44 par ses deux parties rigides 12 et 14. Les deux éléments d'ancrage 42 et 44 sont parallèles entre eux et traversés par un plan axial commun Pa.

Les éléments d'ancrage 42 et 44 présentent une tige filetée 46 surmontée d'une tête 48 formant un U dont la paroi interne est filetée de façon qu'un élément 50 formant vis s'y adapte. Ainsi, les premières portions fixables 16 et 18 des parties rigides 12 et 14 sont encastrées dans les têtes 48 des éléments d'ancrage 42 et 44 respectivement et sont bloquées sur ces derniers par serrage des éléments 50 formant vis.

De la sorte, lorsque les tiges filetées 46 des éléments d'ancrage tendent à se rapprocher sous la contrainte des forces opposées T et -T, contenues dans le plan Pa et sensiblement parallèles à l'axe A, les éléments d'ancrage 42 et 44 déforment la pièce de liaison qui fléchit.

Le fléchissement de la pièce de liaison 10 entraîne la compression du bord inférieur 52 de l'organe de liaison 24 et corrélativement l'extension du bord supérieur 54 diamétralement opposé tandis que les parties rigides 12 et 14 conservent leur forme. Le matériau de l'organe de liaison 24 étant élastiquement déformable, lorsque la contrainte cesse, la pièce de liaison retrouve sa forme initiale rectiligne et les tiges filetées des éléments d'ancrage 46 retrouvent leur position relative.

Le mécanisme de flexion élastique de la pièce de liaison 10 et des éléments d'ancrage 42, 44 décrit ci-dessus est le même lorsque les tiges filetées 46 des éléments d'ancrage 42 et 44 sont respectivement éloignées l'une de l'autre, la pièce de liaison fléchissant selon une courbure inverse.

En outre, les éléments d'ancrage 42 et 44 sont mobiles en translation l'un par rapport à l'autre selon l'axe A, leur déplacement relatif conduisant à l'extension de l'organe de liaison 24 ou à sa compression.

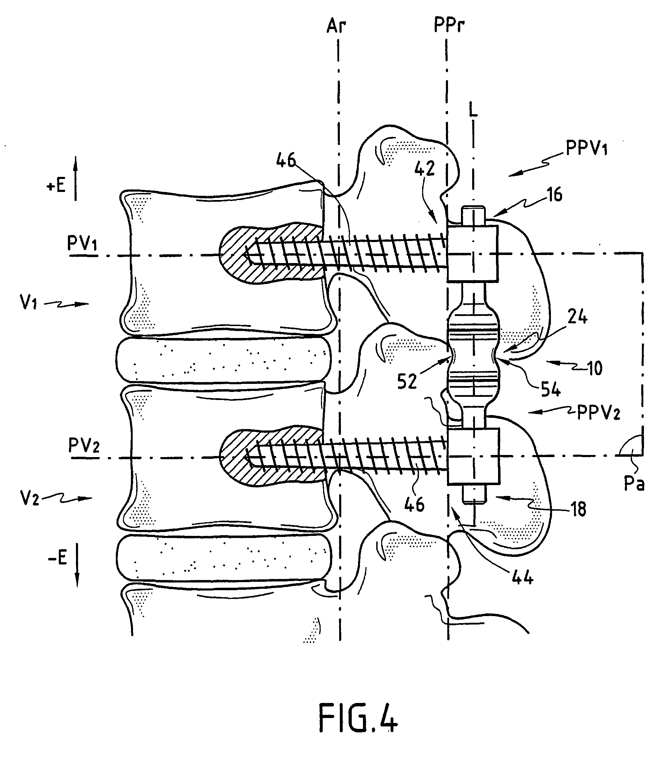

On se référera maintenant à la Figure 4 pour décrire l'utilisation de la pièce de liaison 10 dans un système de stabilisation vertébrale destiné à solidariser au moins deux vertèbres V1 et V2.

Les vertèbres V1 et V2 présentent chacune un plan moyen PV1 et PV2 sensiblement perpendiculaire à l'axe Ar du rachis qu'elles forment et une paroi postérieure PPV1 et PPV2 définissant un plan moyen postérieur PPr dudit rachis.

Le système de stabilisation comporte au moins deux éléments d'ancrage 42 et 44 vissés chacun dans la paroi postérieure PPV1 et PPV2 des vertèbres V1 et V2, respectivement, de façon que la ligne L qui coupe les deux éléments d'ancrage 42 et 44 soit sensiblement parallèle audit axe Ar du rachis. Une pièce de liaison 10 relie les deux éléments d'ancrage 42 et 44 par ses deux premières portions fixables 16 et 18. De la sorte, les vertèbres V1 et V2, reliées dans leur partie postérieure, présentent une mobilité relative selon l'axe Ar du rachis.

Ainsi, lorsque le rachis est mis en extension, les vertèbres V1 et V2 tendent à s'écarter l'une de l'autre selon E et -E respectivement, ce qui entraîne l'écartement des tiges filetées 46 l'une de l'autre également, induisant la déformation de la pièce de liaison 10, et particulièrement de son organe de liaison 24. En effet, ce dernier est comprimé, à la fois longitudinalement et sur le bord supérieur 54. La pièce de liaison ainsi déformée forme une concavité opposée au rachis.

Lorsque le rachis est mis en flexion, l'effet inverse se produit et les vertèbres V1 et V2 tendent à se rapprocher, ce qui induit la déformation de la pièce de liaison de façon à former une concavité dirigée vers le rachis. L'organe de liaison subit alors une extension longitudinale de son bord supérieur 54 et éventuellement une compression de son bord inférieur 52.

On comprend que la pièce de liaison 10 conforme à l'invention procure une plus grande mobilité relative des vertèbres par rapport aux tiges de liaison de l'art antérieur qui ne sont pas susceptibles d'être comprimées longitudinalement.

Selon un mode particulier de réalisation, non représenté, la pièce de liaison comprend trois parties rigides, formant tiges et deux organes de liaison reliant les trois parties rigides. Pour ce faire, la partie rigide centrale comporte une première portion de fixation et deux deuxièmes portions de solidarisation, chacune desdites deuxièmes portions de solidarisation étant située de chaque côté de ladite première portion de fixation, lesdites deuxièmes portions de solidarisation étant reliées auxdits deux organes de liaison. Les deux autres parties rigides, situées aux deux extrémités de ladite pièce, présentent une seule deuxième portion de solidarisation reliée aux organes de liaison.

Ainsi, la pièce de liaison maintient un espacement entre trois éléments d'ancrage qu'elle relie, ces derniers étant fixés dans trois vertèbres sensiblement équidistantes, de façon à constituer un alignement. Chaque partie rigide de la pièce de liaison est fixée à un élément d'ancrage de façon à obtenir un organe de liaison élastiquement déformable entre les vertèbres prises deux à deux. De la sorte, trois vertèbres sont stabilisées avec une seule pièce de liaison unique, ce qui permet de diminuer le temps de montage de l'ensemble du système de stabilisation et par conséquent le temps opératoire. En outre, les trois vertèbres étant reliées par une pièce unique de liaison leur mobilité relative, les unes par rapport aux autres, est mieux contrôlée.

Il va de soi qu'on ne sortirait pas du cadre de l'invention en prévoyant des pièces de liaison comportant plus de trois parties rigides séparées par des organes de liaison élastiquement déformables.

## Revendications

1. Pièce de liaison destinée à maintenir un espacement entre au moins deux éléments d'ancrage vissés dans des vertèbres, comprenant au moins
deux parties rigides formant tiges (12, 14) constituées dans un premier matériau, présentant chacune une première portion de fixation (16, 18) apte à être fixée dans un élément d'ancrage et une deuxième portion de solidarisation (20, 22), lesdites tiges (12, 14) étant situées dans le prolongement l'une de l'autre et lesdites portions de solidarisation (20, 22) en regard l'une de l'autre, et **caractérisée en ce qu'**elle comprend
un organe de liaison (24) intégralement constitué dans un second matériau de plus grande déformabilité élastique que ledit premier matériau, reliant lesdites parties rigides (12, 14) par leurs dites portions de solidarisation (20, 22) en regard l'une de l'autre de façon que ladite pièce de liaison (24) soit apte à être déformée élastiquement, par quoi les vertèbres, maintenues espacées l'une de l'autre, sont mobiles l'une par rapport à l'autre.

2. Pièce de liaison selon la revendication 1, **caractérisée en ce** lesdites parties rigides sont reliées mécaniquement entre elles par un seul organe de liaison réalisant l'intégralité de ladite liaison mécanique.

3. Pièce de liaison selon la revendication 1 ou 2, **caractérisée en ce que** ledit organe de liaison est intégralement constitué dans un seul second matériau.

4. Pièce de liaison selon l'une quelconque des revendications 1 à 3, **caractérisée en ce qu'**elle comprend n parties rigides entre lesquelles sont interposées n-1 organes de liaison selon l'axe longitudinal de ladite pièce, chaque partie rigide située entre deux organes de liaison comportant une première portion de fixation et deux deuxièmes portions de solidarisation, chacune desdites deuxièmes portions de solidarisation étant située de chaque côté de ladite première portion de fixation, lesdites deuxièmes portions de solidarisation étant reliées auxdits deux organes de liaison, et **en ce que** les parties rigides situées aux deux extrémités de ladite pièce présente une seule deuxième portion de solidarisation reliée aux organes de liaison, par quoi ladite pièce est apte à relier n éléments d'ancrage.

5. Pièce de liaison selon l'une quelconque des revendications 1 à 4, **caractérisée en ce que** lesdites portions de solidarisation (20, 22) desdites parties rigides (12, 14) que ledit organe de liaison (24) relie présentent une paroi de solidarisation (20', 22') sur laquelle ledit organe de liaison (24) est apte à adhérer.

6. Pièce de liaison selon la revendication 5, **caractérisée en ce que** ladite paroi de solidarisation (20', 22') présente des évidements (30, 32) aptes à coopérer avec des aspérités (26, 28) dudit organe de liaison (24) de façon à augmenter la surface de contact entre ladite paroi (20', 22') et ledit organe (24).

7. Pièce de liaison selon l'une quelconque des revendications 1 à 6, **caractérisée en ce que** ledit deuxième matériau dudit organe de liaison (24) est constitué d'un corps obtenu par polymérisation.

8. Pièce de liaison selon l'une quelconque des revendications 1 à 7, **caractérisée en ce que** ledit premier matériau desdites parties rigides (12, 14) est un alliage de titane.

9. Pièce de liaison selon l'une quelconque des revendications 1 à 8, **caractérisée en ce que** la section desdites parties rigides (12, 14) formant tiges est circulaire.

10. Système de stabilisation vertébrale destiné à solidariser au moins deux vertèbres (V1, V2), lesdites vertèbres présentant chacune un plan moyen (PV1, PV2) sensiblement perpendiculaire à l'axe du rachis (Ar) qu'elles forment et une paroi postérieure (PPV1, PPV2) définissant un plan moyen postérieur (PPr) dudit rachis, ledit système comportant au moins deux éléments d'ancrage (42, 44) aptes à être fixés chacun dans la paroi postérieure (PPV1, PPV2) d'une vertèbre de façon que la ligne qui coupe lesdits deux éléments d'ancrage (42, 44) soit sensiblement parallèle audit axe du rachis (Ar),
**caractérisé en ce qu'**il comprend au moins une pièce de liaison (10) selon l'une quelconque des revendications 1 à 9 apte à relier lesdits deux éléments d'ancrage (42, 44) par lesdites deux parties rigides (12, 14) de façon que l'axe de ladite pièce de liaison (A) soit sensiblement parallèle audit axe du rachis (Ar), par quoi lesdites vertèbres (V1, V2), reliées dans leur partie postérieure, présentent une mobilité relative selon ledit axe dudit rachis (Ar).

## Patentansprüche

1. Verbindungsstück zum Halten eines Abstands zwischen wenigstens zwei in Wirbeln verschraubten Verankerungselementen, das wenigstens aufweist:
zwei Stangen bildende steife Teile (12, 14), die aus einem ersten Material hergestellt sind, die jeweils einen ersten Befestigungsabschnitt (16, 18), der in einem Verankerungselement befestigt werden kann und einen zweiten Verbindungsabschnitt (20, 22) aufweisen, wobei die Stangen (12, 14) in der Verlängerung voneinander liegen und die Verbindungsabschnitte (20, 22) einander gegenüber liegend angeordnet sind, **dadurch gekennzeichnet, dass** es ein Verbindungselement (24) aufweist, das vollständig aus einem zweiten Material mit größerer elastischer Verformbarkeit als das erste Material gebildet ist, das die steifen Teile (12, 14) durch ihre Verbindungsabschnitte (20, 22) einander gegenüber verbindet, derart, dass das Verbindungsstück (24) elastisch verformt werden kann, wodurch die Wirbel, die im Abstand zueinander gehalten werden, jeweils gegeneinander beweglich sind.

2. Verbindungsstück nach Anspruch 1, **dadurch gekennzeichnet, dass** die steifen Teile mechanisch untereinander durch ein einziges Verbindungselement verbunden sind, das die Vollständigkeit der mechanischen Verbindung herstellt.

3. Verbindungsstück nach Anspruch 1 oder 2, **dadurch gekennzeichnet, dass** das Verbindungselement vollständig aus einem einzigen zweiten Material gebildet ist.

4. Verbindungsstück nach einem der Ansprüche 1 bis 3, **dadurch gekennzeichnet, dass** es n steife Teile aufweist, zwischen denen n-1 Verbindungselemente entlang der Längsachse des Stücks eingefügt sind, wobei jedes zwischen zwei Verbindungselementen liegende steife Teil einen ersten Befestigungsabschnitt und zwei zweite Verbindungsabschnitte aufweist, wobei jeder der zweiten Verbindungsabschnitte auf jeder Seite des ersten Befestigungsabschnitts liegt, wobei die zweiten Verbindungsabschnitte mit den beiden Verbindungselementen verbunden sind, und dass die an den beiden Enden des Stücks liegenden, steifen Teile einen einzigen zweiten Verbindungsabschnitt aufweisen, der mit den Verbindungselementen verbunden ist, wodurch das Stück n Verankerungselemente verbinden kann.

5. Verbindungsstück nach einem der Ansprüche 1 bis 4, **dadurch gekennzeichnet, dass** die Verbindungsabschnitte (20, 22) der steifen Teile (12, 14), die das Verbindungselement (24) verbindet, eine Verbindungswand (20', 22') aufweisen, auf der das Verbindungselement (24) haften kann.

6. Verbindungsstück nach Anspruch 5, **dadurch gekennzeichnet, dass** die Verbindungswand (20', 22') Aussparungen (30, 32) aufweist, die mit Unebenheiten (26, 28) des Verbindungselements (24) zusammenwirken können, so dass die Kontaktfläche zwischen der Wand (20', 22') und dem Element (24) vergrößert wird.

7. Verbindungsstück nach einem der Ansprüche 1 bis 6, **dadurch gekennzeichnet, dass** das zweite Material des Verbindungselements (24) aus einem durch Polymerisation erhaltenen Körper gebildet ist.

8. Verbindungsstück nach einem der Ansprüche 1 bis 7, **dadurch gekennzeichnet, dass** das erste Material der steifen Teile (12, 14) eine Titanlegierung ist.

9. Verbindungsstück nach einem der Ansprüche 1 bis 8, **dadurch gekennzeichnet, dass** der Querschnitt der die Stangen bildenden steifen Teile (12, 14) kreisförmig ist.

10. System zur vertebralen Stabilisierung, das zum Verbinden von wenigstens zwei Wirbeln (V1, V2) bestimmt ist, wobei die Wirbel jeweils eine mittlere Ebene (PV1, PV2), die im Wesentlichen senkrecht zur Achse der Wirbelsäule (Ar), die sie bilden, verläuft, und eine Hinterwand (PPV1, PPV2) aufweisen, die eine hintere mittlere Ebene (PPr) der Wirbelsäule definiert, wobei das System wenigstens zwei Verankerungselemente (42, 44) aufweist, die jeweils in der Hinterwand (PPV1, PPV2) eines Wirbels befestigt werden können, derart, dass die Linie, die die beiden Verankerungselemente (42, 44) schneidet, im Wesentlichen parallel zu der Achse der Wirbelsäule (Ar) verläuft,
**dadurch gekennzeichnet, dass** es wenigstens ein Verbindungsstück (10) nach einem der Ansprüche 1 bis 9 aufweist, das die beiden Verankerungselemente (42, 44) durch die beiden steifen Stücke (12, 14) verbinden kann, derart, dass die Achse des Verbindungsstücks (A) im Wesentlichen parallel zur Achse der Wirbelsäule (Ar) verläuft, wodurch die Wirbel (V1, V2), die in ihrem Hinterteil verbunden sind, eine Beweglichkeit jeweils entlang der Achse der Wirbelsäule (Ar) aufweisen.

## Claims

1. A connecting member for maintaining the spacing between at least two anchor members screwed into vertebrae, including at least two rigid rod-forming parts (12, 14) made of a first material and each having a fixing, first portion (16, 18) adapted to be fixed into an anchor member and a fastening, second portion (20, 22), said rods (12, 14) being aligned with each other and said fastening portions (20, 22) facing each other, and **characterised in that** it includes a connecting body (24) that is made entirely from a second material that is more elastically deformable than said first material and interconnects the facing fastening portions (20, 22) of said rigid parts (12, 14) so that said connecting body (24) is able to deform elastically, whereby the vertebrae, which are held spaced from each other, are movable relative to each other.

2. A connecting member according to claim 1, **characterised in that** said rigid parts are mechanically connected together by a single connecting body providing the whole of said mechanical connection.

3. A connecting member according to claim 1 or claim 2, **characterised in that** said connecting body is entirely made of a second material.

4. A connecting member according to any one of claims 1 to 3, **characterised in that** it includes n rigid parts with n-1 connecting bodies disposed between them along the longitudinal axis of said member, each rigid part situated between two connecting bodies having one fixing, first portion and two fastening, second portions, there being one fastening, second portion at each end of said fixing, first portion, and said fastening, second portions being connected respectively to said two connecting bodies, and **in that** the rigid parts at the two ends of said member have a single fastening, second portion connected to the connecting bodies, whereby said connecting member is adapted to connect n anchor members.

5. A connecting member according to any one of claims 1 to 4, **characterised in that** said fastening portions (20, 22) of said rigid parts (12, 14) connected by said connecting body (24) have a fastening wall (20', 22') to which said connecting body (24) is adapted to adhere.

6. A connecting member according to claim 5, **characterised in that** said fastening wall (20', 22') has openings (30, 32) adapted to cooperate with asperities (26, 28) on said connecting body (24) to increase the contact area between said wall (20', 22') and said connecting body (24).

7. A connecting member according to any one of claims 1 to 6, **characterised in that** said second material of which said connecting body (24) is made is obtained by polymerisation.

8. A connecting member according to any one of claims 1 to 7, **characterised in that** said first material of which said rigid parts (12, 14) are made is a titanium alloy.

9. A connecting member according to any one of claims 1 to 8, **characterised in that** the section of said rigid rod-forming parts (12, 14) is circular.

10. A vertebral stabilization system for fastening together at least two vertebrae (V1, V2) each having a median plane (PV1, PV2) substantially perpendicular to the axis (Ar) of the spine of which they are part and a posterior wall (PPV1, PPV2) defining a posterior median plane (PPr) of said spine, said system comprising at least two anchor members (42, 44) each adapted to be fixed to the posterior wall (PPV1, PPV2) of a vertebra so that a line which intersects said two anchor members is substantially parallel to said axis (Ar) of the spine,
the system being **characterised in that** it further comprises at least one connecting member (10) according to any one of claims 1 to 9 whose two rigid parts (12, 14) are adapted to interconnect said two anchor members (42, 44) so that the axis (A) of said connecting member is substantially parallel to said axis (Ar) of the spine, whereby said vertebrae (V1, V2), which are interconnected via their posterior portions, present relative mobility along said axis (Ar) of said spine.
